# FASCICULE DE BREVET EUROPEEN

(11) **EP 0 856 309 B1**
(45) Date de publication et mention de la délivrance du brevet: **27.10.1999**
(21) Numéro de dépôt: 98400013.3
(22) Date de dépôt: 06.01.1998
(51) Int. Cl.: A61K 7/48

(54) **Emulsion eau-dans-huile, composition comprenant une telle émulsion et utilisation en cosmétique, en pharmacie ou en hygiène**
Wasser-in-Öl Emulsion, diese enthaltende Zusammensetzung, und ihre Verwendung in der Kosmetik, in der Pharmazie oder in der Hygiene
Water-in-oil emulsion, composition comprising it and its use in cosmetics, pharmacy or hygiene

(30) Priorité: 24.01.1997 FR 9700914
(43) Date de publication de la demande: 05.08.1998
(73) Titulaire: L'OREAL, 75008 Paris (FR)
(72) Inventeur: Terren, Nadia, 94550 Chevilly-Larue (FR); Fontaine, Jacqueline, 78170 La Celle Saint Cloud (FR)
(74) Mandataire: Lhoste, Catherine

(56) Documents cités:
- EP-A- 0 391 274
- EP-A- 0 655 234
- WO-A-95/03778
- WO-A-96/33689
- FR-A- 2 439 798
- FR-A- 2 679 769
- GB-A- 2 144 133

## Description

L'invention concerne une émulsion eau-dans-huile comprenant un polymère dispersé dans la phase aqueuse, et son application en cosmétique, en pharmacie ou en hygiène.

Les compositions de fonds de teint se présentent généralement sous forme de crème plus ou moins fluide comprenant des corps gras tels que des huiles et une phase particulaire généralement composée de charges et de pigments. Ces compositions, lorsqu'elles sont appliquées sur la peau, présentent toutefois l'inconvénient de transférer, c'est-à-dire de se déposer au moins en partie, en laissant une trace, sur certains supports avec lesquels elles peuvent être mises en contact, et notamment un vêtement ou la peau. Il s'en suit une persistance médiocre du film sur la peau d'où la nécessité de renouveler régulièrement l'application de la composition de fond de teint.
Un autre inconvénient des compositions de l'art antérieur réside dans le problème de migration de ces compositions, c'est-à-dire dans le fait que la composition a tendance à se propager à l'intérieur des plis et/ou des rides du visage, en créant un effet inesthétique.

Il est connu, selon la demande de brevet WO-A-96/33689, une composition de fond de teint sous forme d'émulsion eau-dans-huile, la phase grasse comprenant des huiles volatiles et un polymère filmogène étant dispersé dans la phase aqueuse. Parmi les polymères préconisés pour cette composition sont mentionnées les résines acryliques comme le DERMACRYL LT.
Or, on a constaté que l'addition d'un tel polymère dans la phase aqueuse présente l'inconvénient de déstabiliser l'émulsion au cours du temps. En effet, après stockage l'émulsion ne conserve pas son homogénéité et une démixtion de l'émulsion apparaît rapidement, néfaste aux propriétés requises pour la composition.

Le but de la présente invention est de fournir une émulsion eau-dans-huile qui présente une bonne stabilité, tout en conservant de bonnes propriétés cosmétiques. En particulier, il est recherché de pouvoir disposer d'une émulsion eau-dans-huile stable qui ne transfère pas après son application, notamment sur la peau.

Le demandeur a découvert de façon surprenante et inattendue qu'en utilisant un polymère particulier dispersé dans la phase aqueuse de l'émulsion, en association avec un choix d'huiles particulier, on peut obtenir une émulsion eau-dans-huile présentant lesdites caractéristiques, et qui présente également l'avantage de ne pas transférer et d'être stable.

La présente invention a donc pour objet une émulsion eau-dans-huile comprenant une phase grasse comprenant au moins une huile volatile, la phase aqueuse comprenant un polymère filmogène en dispersion dans ladite phase aqueuse, caractérisée par le fait que le polymère filmogène est un copolymère comprenant au moins un monomère acide carboxylique comportant au moins une insaturation éthylénique et au moins un ester d'acide carboxylique dont le groupement lié à l'atome d'oxygène de la fonction ester comporte au moins une insaturation éthylénique.
Un autre objet de l'invention concerne une composition, en particulier cosmétique, pharmaceutique ou hygiénique, comprenant une émulsion telle que définie ci-dessus.
L'invention porte également sur un procédé de traitement non thérapeutique de la peau et/ou du cuir chevelu, notamment un procédé de maquillage, consistant à appliquer sur la peau et/ou sur le cuir chevelu une émulsion et/ou une composition telles que définies ci-dessus.
Un autre objet de l'invention concerne l'utilisation dudit polymère dispersé dans la phase aqueuse de ladite émulsion eau-dans-huile dans une composition dite 'sans transfert' et/ou pour diminuer le transfert et/ou la migration d'une composition la comprenant, sur la peau.

On a également constaté que l'émulsion utilisée selon l'invention s'applique et s'étale facilement de façon homogène, sans laisser de sensation de gras et présente de bonnes propriétés cosmétiques. Le film obtenu présente également une texture légère et reste confortable à porter tout au long de la journée.
De plus, l'émulsion appliquée sur la peau présente l'avantage de ne pas migrer dans les plis de la peau et/ou les rides du visage.
Par ailleurs, il est possible d'ajouter à l'émulsion selon l'invention d'autres additifs tels que des huiles et/ou des poudres (pigments et/ou charges) tout en conservant une émulsion stable. L'émulsion est donc compatible avec un grand nombre d'adjuvants cosmétiques.
Enfin, on a constaté que la viscosité de l'émulsion est stable dans le temps.

Ainsi, selon l'invention, le polymère filmogène dispersé dans la phase aqueuse de l'émulsion est un copolymère comprenant au moins un monomère acide carboxylique comportant au moins une insaturation éthylénique et au moins un ester d'acide carboxylique dont le groupement lié à l'atome d'oxygène de la fonction ester comporte au moins une insaturation éthylénique.

Selon une variante de réalisation de l'invention, le polymère filmogène peut comprendre en outre au moins un ester d'acide carboxylique de type acrylate.

Avantageusement, le polymère filmogène dispersé dans la phase aqueuse peut être choisi parmi :
- les copolymères consistant en au moins un monomère acide carboxylique comportant au moins une insaturation éthylénique et au moins un ester d'acide carboxylique dont le groupement lié à l'atome d'oxygène de la fonction ester comporte au moins une insaturation éthylénique, et
- les copolymères consistant en au moins un monomère acide carboxylique comportant au moins une insaturation éthylénique, au moins un ester d'acide carboxylique dont le groupement lié à l'atome d'oxygène de la fonction ester comporte au moins une insaturation éthylénique, et au moins un ester d'acide carboxylique de type acrylate.

Parmi les polymères filmogènes radicalaires à fonctions acides carboxyliques utilisables selon l'invention, on peut notamment citer :
- le copolymère acétate de vinyle/acide crotonique (90/10) vendu par la société BASF sous la dénomination de "Luviset CA66",
- le terpolymère acétate de vinyle/acide crotonique/néodécanoate de vinyle vendu par la Société National Starch sous la dénomination de "Résine 28-29-30". On peut également utiliser selon l'invention les polymères filmogènes ayant la formule générale (I) suivante : 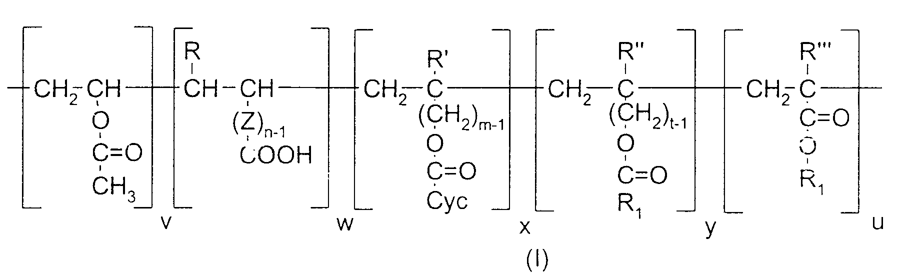 dans laquelle :
- R, R', R'' et R''', identiques ou différents, représentent un atome d'hydrogène ou un radical méthyle,
- m, n et t sont 1 ou 2,
- R1 représente un radical alkyle, linéaire ou ramifié, saturé ou insaturé ayant de 2 à 21 atomes de carbone,
- Z représente un radical divalent pris dans le groupe constitué par :
   -CH2-, -CH2-O-CH2- et -CH2-O-(CH2)2-,
- Cyc représente un radical choisi parmi 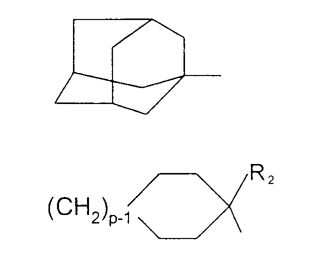 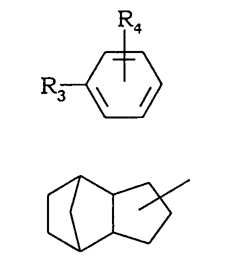 dans lesquels
- R2 représente un atome d'hydrogène ou un radical méthyle,
- R3 représente un atome d'hydrogène, un radical méthyle, éthyle, tert-butyle, éthoxy, butoxy ou dodécyloxy,
- R4 représente un atome d'hydrogène, un radical alkyle de 1 à 4 atomes de carbone ou un radical alkoxy de 1 à 4 atomes de carbone,
- p est égal à 1 ou 2
- v représente de 10 à 97 % et de préférence de 36 à 84% en poids,
- w représente de 3 à 20 % et de préférence de 6 à 12% en poids,
- x représente de 0 à 60 %, de préférence de 4 à 60 %, et plus préférentielle-ment de 6 à 40% en poids,
- y représente de 0 à 40% et de préférence de 4 à 30% en poids,
- u représente de 0 à 30 % en poids et de préférence de 0 à 20 % en poids,
- v + w + x + y + u étant égal à 100%.

Des polymères filmogènes de formule (I) sont décrits dans la demande de brevet FR-A- 2.439.798.
La dispersion du polymère filmogène dans la phase aqueuse de l'émulsion selon l'invention peut être obtenue par simple addition d'un latex ou d'un pseudolatex dudit polymère filmogène aux autres ingrédients de la phase aqueuse de l'émulsion, notamment lors de la préparation de l'émulsion.

Les latex résultent directement de la synthèse d'un polymère par une technique bien connue de polymérisation en émulsion. La neutralisation éventuelle du latex est telle que le polymère reste sous forme de latex et ne se solubilise pas dans l'eau.
Pour l'obtention d'un pseudolatex, on prépare un polymère et on le met ensuite en dispersion dans l'eau. La dispersion dans l'eau est autostabilisée par neutralisation au moins partielle des groupes acides portés par le polymère. Des dispersions aqueuses sous forme de pseudolatex des polymères filmogènes cités précédemment, et notamment des polymères de formule (I), sont décrites dans les demandes de brevet EP-A-618793, EP-A-628304 et EP-A-679384.

Plus préférentiellement, on utilise le copolymère acétate de vinyle/acide crotonique/tert-butyl-4 benzoate de vinyle (65/10/25).

Avantageusement, on neutralise au moins partiellement les fonctions acides carboxyliques des polymères filmogènes, pour obtenir une bonne dispersion du polymère filmogène dans le milieu aqueux.
Le taux de neutralisation des polymères filmogènes à fonctions acides carboxyliques doit être déterminé de telle sorte qu'ils restent insolubles dans l'eau tout en étant solubles dans le solvant organique. Ce taux de neutralisation doit donc être inférieur à 100%.
Les taux limites inférieur et supérieur de neutralisation qu'il convient de ne pas dépasser pour que le polymère reste insoluble dans l'eau sont fonctions de la nature de chaque polymère et sont aisément déterminables par l'homme du métier sur la base de ses connaissances techniques générales.
De façon générale, le taux de neutralisation est compris entre 30 et 80%, si le polymère a moins de 2meq/g de fonctions acides carboxyliques, et entre 10 et 50% si le polymère a plus de 2meq/g de fonctions acides carboxyliques.

La neutralisation des fonctions acides carboxyliques peut être réalisée à l'aide d'un composé basique, tel qu'une base minérale comme la soude ou la potasse, ou un aminoalcool par exemple pris dans le groupe constitué par l'amino-2-méthyl-2-propanol-1 (AMP), la triéthanolamine, la triisopropanolamine (TIPA), la monoéthanolamine, la diéthanolamine, la tri-[hydroxy-2-propyl-1]-amine, l'amino-2-méthyl-2-propanediol-1,3 (AMPD) et l'amino-2-hydroxyméthyl-2-propanediol-1,3. On peut également utiliser la lysine, l'arginine ou la cystine comme agent neutralisant.

La taille moyenne des particules de polymère filmogène est généralement comprise entre 10 et 300 nm, de préférence inférieure à 250 nm, avec une polydispersité en taille des particules relativement faible.

Afin d'obtenir de bonnes propriétés filmogènes du polymère dispersé dans la phase aqueuse de l'émulsion selon l'invention, il peut être avantageux d'ajouter avec le polymère filmogène au moins un agent plastifiant. Cet agent plastifiant peut être hydrophile ou hydrophobe. Il est choisi de manière à permettre, en association avec le polymère considéré, un film souple et non friable. On peut introduire l'agent plastifiant en mélange dans le solvant organique, lors de la préparation de la dispersion aqueuse, notamment lorsqu'il est du type hydrophobe. Lorsqu'il est du type hydrophile, on peut l'introduire directement dans la dispersion après sa formation.
Parmi les agents plastifiants pouvant être utilisés dans la présente invention, on peut citer:
- les Carbitols de la Société Union Carbide à savoir le Carbitol ou diéthylène glycol éthyléther, le méthyl Carbitol ou diéthylène glycol méthyléther, le butyl Carbitol ou diéthylène glycol butyléther ou encore l'hexyl Carbitol ou diéthylène glycol hexyléther,
- les Cellosolves de la Société Union Carbide à savoir le Cellosolve ou éthylène glycol éthyléther, le butyl Cellosolve ou éthylène glycol butyléther, l'hexyl Cellosolve ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les Dowanols de la Société Dow Chemical tels que le Dowanol PM ou propylène glycol méthyléther, le Dowanol DPM ou dipropylène glycol éthyléther, le Dowanol TPM ou tripropylène glycol méthyléther et le Dowanol DM ou diéthylène glycol méthyléther,
- l'alcool benzylique,
- le citrate de triéthyle,
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2-hexyle, et
- les esters de glycérol tels que le diacétate de glycérol (diacétine) et le triacétate de glycérol (triacétine).
On introduit généralement 5 à 60 % d'agent plastifiant par rapport au poids de matière sèche de polymère filmogène neutralisé, ledit agent étant distribué selon son coefficient de partage entre lesdites particules et la phase aqueuse.

Selon l'invention, le polymère filmogène en dispersion dans la phase aqueuse de l'émulsion peut être présent en une teneur allant de 0,3 % à 10 % en poids, et de préférence de 2 % à 5 %, par rapport au poids total de l'émulsion.

Avantageusement, l'émulsion selon l'invention est réalisée à l'aide d'un tensioactif émulsionnant convenant pour la préparation d'une émulsion eau-dans-huile stable, c'est-à-dire ayant un HLB permettant d'obtenir une émulsion eau-dans-huile. Par exemple, le tensioactif peut être un tensioactif siliconé, et notamment une silicone oxyalkylénée.
Selon l'invention, on désigne par silicone oxyalkylénée toute silicone comportant au moins un groupement oxyalkyléné de type (-CₓH₂ₓO)ₐ dans lequel x peut varier de 2 à 6 et a est supérieur ou égal à 1.
Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle.

Les silicones oxyalkylénées sont par exemple choisies parmi les composés de formules générales (VII), (VIII), (IX), et (X) : 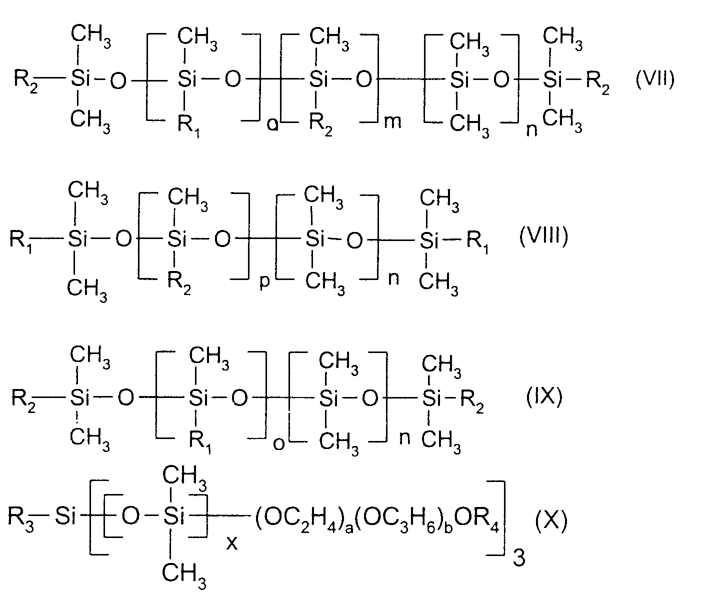 formules dans lesquelles :
- R₁, identique ou différent, représente un radical alkyle, linéaire ou ramifié, en C₁-C₃₀ ou phényle.
- R₂, identique ou différent, représente un radical
   -C_{c}H_{2c}-O-(C₂H₄O)ₐ(C₃H₆O)_{b}-R₅ ou un radical -C_{c}H_{2c}-O-(C₄H₈O)ₐ-R₅,
- R₃, R₄, identiques ou différents, désignent un radical alkyle, linéaire ou ramifié, en C₁-C₁₂, et de préférence le radical méthyle,
- R₅, identique ou différent, est choisi parmi un atome d'hydrogène, un radical alkyle, linéaire ou ramifié, de 1 à 12 atomes de carbone, linéaire ou ramifié, de 1 à 6 atomes de carbone, un radical acyle, linéaire ou ramifié, de 2 à 12 atomes de carbone, un radical hydroxyle, -SO₃M, -OCOR₆, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement phosphono éventuellement substitué par un ou deux radicaux aminoalkyle substitués, -CO(CH₂)_{d}COOM, -OCOCHR₇(CH₂)_{d}COOM, -NHCO(CH₂)_{d}OH, -NH₃Z,
- M, identique ou différent, désigne un atome d'hydrogène, Na, K, Li, NH₄ ou une amine organique,
- R₆ désigne un radical alkyle, linéaire ou ramifié, en C₁ -C₃₀,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 1,
- c varie de 0 à 4,
- x varie de 1 à 100,
- Z représente un anion minéral ou organique monovalent tel que halogénure (chlorure, bromure), sulfate, carboxylate (acétate, lactate, citrate).

De préférence, on utilise les silicones oxyalkylénées répondant aux formules générales (VII) ou (VIII). Plus particulièrement, ces formules répondent à au moins une des, et de préférence toutes les, conditions suivantes :
- c est égal à 2 ou 3.
- R₁ désigne le radical méthyle.
- R₅ représente un atome d'hydrogène, un radical méthyle ou un radical acétyle et de préférence un atome d'hydrogène.
- a varie de 1 à 25 et plus particulièrement de 2 à 15.
- b est égal à 0.
- n varie de 0 à 100.
- p varie de 1 à 20

On peut également utiliser des silicones oxyalkylénées de formule (XI) suivante : 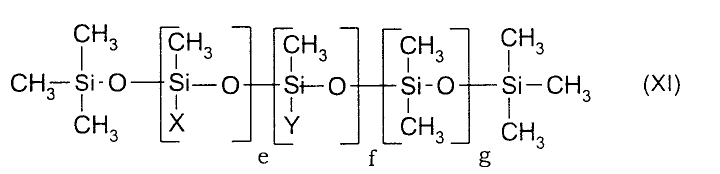 dans laquelle :
X désigne un groupement -(CH2)₃-O-(C₂H₄O-)ₓ-(C₃H₆O-)_{y}-W
W est un atome d'hydrogène, un alkyle en C1-C16, un acyle en C1-C16,
Y est un radical alkyle en C8-C22 ou acyle en C8-C22,
g = 0 à 200,
e = 1 à 40,
f = 1 à 100,
   le poids moléculaire du reste -(C₂H₄O-)ₓ-(C₃H₆O-)_{y}-W est de 250 à 2000, x et y étant choisis de telle sorte que le rapport en poids des groupes oxyéthylène/ oxypropylène soit compris entre 1000:0 et 20:80.

Des tensioactifs siliconés ayant un HLB permettant d'obtenir des émulsions eaudans-huile sont par exemples vendus par la société GOLDSCHMIDT sous les dénominations commerciales ABIL WE 09, ABIL EM 90, ABIL B8852, par la société DOW CORNING sous les dénominations DC 3225 C, Q2-5220, Q2-5200, par la société AMERCHOL sous la dénomination SILSOFT BEAUTY AID SL.

Avantageusement, on utilise un tensioactif siliconé de formule (XI) et plus préférentiellement ceux pour lesquels W est un atome d'hydrogène, Y est un radical alkyle en C8-C22.
Le tensioactif utilisé selon l'invention peut être présent dans l'émulsion en une teneur allant de 5 % à 12 % en poids, et de préférence de 7 à 10 % en poids, par rapport au poids total de l'émulsion.
L'émulsion selon l'invention comprend également, dans une phase grasse, au moins une huile volatile à une teneur d'au moins 65 % en poids par rapport au poids total de la phase grasse.

Par huile volatile, on entend dans la présente description, toute huile susceptible de s'évaporer au contact de la peau.
De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100 °C, et/ou dont la pression de vapeur, mesurée à 10⁵ Pa et à 25 °C, est supérieure ou égale à 0,02 mm Hg (2,6 Pa) et/ou dont le point d'ébullition, mesuré à 10⁵ Pa est inférieur ou égal à 275 °C.
L'huile volatile présente dans la phase grasse peut être choisie parmi les huiles volatiles hydrocarbonées, les huiles volatiles siliconées et leurs mélanges.
Parmi les huiles volatiles hydrocarbonées, on peut citer les isoparaffines et notamment l'isododécane.
Parmi les huiles siliconées volatiles, on peut citer :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane.

La phase grasse de l'émulsion selon l'invention peut comprendre, en plus de l'huile volatile, d'autres corps gras non volatils usuellement utilisés dans le domaine d'application envisagé. De préférence, la phase grasse comprend de 65 % à 99 % en poids, de préférence de 75 % à 98 %, par rapport au poids total de la phase grasse, d'huile volatile, siliconée et/ou hydrocarbonées, et de 1 % à 35 % , de préférence de 2 % à 25 % en poids de corps gras non volatils.

Parmi les corps gras non volatils, on peut citer les huiles non volatiles, les corps gras pâteux, les gommes et les cires végétales, minérales, animales et/ou synthétiques, ces derniers comprenant les corps gras siliconés. On peut définir les composés gras pâteux à l'aide d'au moins une des propriétés physico-chimiques suivantes :
- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.
Parmi les corps gras siliconés, on peut citer les polyalkyl(C₁-C₂₀)siloxanes, les huiles de silicone phénylées, ainsi que les gommes de silicones et les cires de silicone.
Parmi les corps gras non siliconés, on peut citer l'huile de paraffine, de vaseline, le perhydrosqualène, l'huile d'abricot, l'huile de germes de blé, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides; des huiles hydrogénées concrètes à 25°C; des lanolines; des esters gras concrets à 25°C; la cire d'abeilles; les cires végétales telles que la cire de Carnauba, de Candellila, d'Ouricury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

De préférence, la phase grasse comprend au moins 75% en poids, par rapport au poids total de ladite phase grasse, d'huile volatile, siliconée et/ou hydrocarbonée. Avantageusement, ladite phase grasse de l'émulsion comprend 100 % en poids, par rapport au poids total de la phase grasse, d'huile volatile.

Les polyalkylsiloxanes selon l'invention sont à groupements terminaux triméthylsilyle. On peut utiliser de préférence ceux dont la viscosité à 25°C est inférieure ou égale à 0,06 m²/s, parmi lesquels on peut citer :
- les polydiméthylsiloxanes linéaires et notamment ceux vendus sous les dénominations "DOW CORNING Fluid 200" par la société DOW CORNING
- les alkylméthylpolysiloxanes tels que la cétyldiméthicone (nom CTFA).

Les gommes de silicone peuvent répondre à la formule : 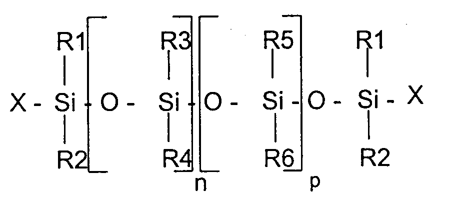 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle et notamment phényle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.
De manière générale, n et p peuvent prendre des valeurs de 0 à 5000, de préférence de 0 à 3000.

Comme gomme de silicone utilisable selon l'invention, on peut citer celles pour lesquelles :
- les substituants R1 à R6 et X représentent un groupement méthyle, p = 0 et n = 2700, comme celle vendue sous la dénomination SE30 par la société General Electric,
- les substituants R1 à R6 et X représentent un groupement méthyle, p = 0 et n = 2300, comme celle vendue sous la dénomination AK 500000 par la société Waker,
- les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
- les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
- les substituants R1, R2, R5, R6 et X représentent un groupement méthyle, les substituants R3 et R4 représentent un groupement phényle tel que le poids moléculaire du composé soit de 600 000, comme celle vendue sous les dénominations "761" ou "MIRASIL C-DPDM" par la société Rhône-Poulenc.

De manière préférentielle, on utilise comme autre corps gras les gommes de silicones.
Ces corps gras peuvent en particulier être choisis de manière variée par l'homme du métier afin de préparer une composition ayant les propriétés souhaitées, par exemple en consistance ou en texture. Ils sont de préférence utilisés à une teneur inférieure ou égale à 7 % en poids par rapport au poids total de l'émulsion, afin de conserver les propriétés avantageuse de l'émulsion utilisée selon l'invention.

La phase aqueuse de l'émulsion selon l'invention peut comprendre de l'eau ou une eau florale telle que l'eau de bleuet.
En outre, la phase aqueuse peut comprendre de 0 % à 14 % en poids, par rapport au poids total de la phase aqueuse, d'un monoalcool inférieur en C₂-C₆ et/ou d'un polyol tel que le glycérol, le butylèneglycol, l'isoprèneglycol, le propylèneglycol.

D'une manière générale, l'émulsion selon l'invention peut comprendre de 30 % à 55 % en poids de phase grasse, de 5 % à 12 % en poids de tensioactif, et de 35 % à 65 % en poids de phase aqueuse.

Par ailleurs, l'émulsion selon l'invention peut comprendre de 0 à 5 % en poids, par rapport au poids total de l'émulsion, d'au moins un co-émulsionnant qui peut être choisi parmi des esters d'acides gras tels que le stéarate de glycéryle, le stéarate d'éthylène glycol acétylé, le succinate d'isostéaryl de diglycéryle, l'isostéarate de sorbitane.

En outre, l'émulsion selon l'invention peut comprendre un ou plusieurs agents épaississants dans des concentrations préférentielles allant de 0 à 6 % en poids, par rapport au poids total de l'émulsion.
L'agent épaississant peut être choisi parmi les argiles modifiées telles que le silicate de magnésium modifié (bentone gel VS38 de RHEOX), l'hectorite modifiée par le chlorure de distéaryl diméthyl ammonium (bentone 38 CE de RHEOX).

L'émulsion selon l'invention peut également comprendre une phase particulaire qui peut comprendre des pigments et/ou des nacres et/ou des charges habituellement utilisés dans les compositions cosmétiques.
Les pigments peuvent être présents dans l'émulsion à raison de 0-20 % en poids, par rapport au poids total de l'émulsion, et de préférence à raison de 2-15 %. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré. Parmi les pigments organiques, on peut citer le noir de carbone, et les laques de baryum, strontium, calcium, aluminium. Les pigments peuvent également présenter une surface hydrophobe ou peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères, notamment des polyéthylènes et/ou des acides aminés.
Les charges, qui peuvent être présentes dans l'émulsion à raison de 0-20 % en poids, par rapport au poids total de l'émulsion, de préférence 0-10 %, peuvent être minérales ou de synthèse, lamellaires ou non lamellaires. On peut citer le talc, le mica, la silice, le kaolin, le Téflon, l'amidon, la nacre naturelle, le nitrure de bore, les microsphères telles que l'Expancel (Nobel Industrie), le polytrap (Dow Corning). De préférence, on utilise des charges sphériques dont la taille est inférieure à 25 µm telles que les poudres de polyéthylène, les poudres de Nylon, les microbilles de résine de silicone (Tospearls de Toshiba), les microsphères de silice, ces charges pouvant contribuer à améliorer les propriétés non transfert des émulsions de l'invention.

L'émulsion selon l'invention peut comprendre en outre un milieu cosmétiquement, pharmaceutiquement ou hygiéniquement acceptable. Elle peut comprendre alors tout additif usuellement utilisé dans le domaine cosmétique, pharmaceutique ou hygiénique, tels que des antioxydants, des colorants, des parfums, des huiles essentielles, des conservateurs, des actifs cosmétiques, des hydratants, des vitamines, des sphingolipides, des polymères liposolubles notamment hydrocarbonés, tels que le polybutène, les polyalkylènes, les polyacrylates et les polymères siliconés compatibles avec les corps gras. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée. Ces additifs peuvent être présents dans la composition à raison de 0-10% en poids.

Les émulsions selon l'invention peuvent se présenter sous la forme d'un produit cosmétique et notamment sous la forme d'un produit de soin pour le corps et/ou le visage et/ou le cuir chevelu ou bien encore d'un produit de maquillage, en particulier un fond de teint, un fard à joues ou à paupières, un eye-liner, un mascara ou un rouge à lèvres.

Elles peuvent également se présenter sous forme non colorée, contenant éventuellement des actifs cosmétiques. L'émulsion selon l'invention peut être sous forme d'une crème, d'un lait ou d'un sérum, susceptible d'être utilisé comme produit de soin ou solaire.

Les exemples qui suivent servent à illustrer l'invention sans toutefois présenter un caractère limitatif.

### Exemple 1:

On a préparé une émulsion eau-dans-huile ayant la composition suivante :

### Phase grasse:

| | |
|---|---|
| - Mélange de polydiphényl diméthylsiloxane (PM = 600000) et de cyclodiméthylsiloxane (15/85) vendu sous la dénomination MIRASIL C-DP DM par la société RHÔNE-POULENC | 8 g |
| - Coémulsionnant | 0,5 g |
| - cyclopentadiméthylsiloxane | 14 g |
| - isododécane | 5 g |
| - Pigments | 8 g |
| - Poudre de nylon | 8 g |
| - Agent épaississant | 1,6 g |

### Phase aqueuse:

| | |
|---|---|
| - Dispersion aqueuse du copolymère acétate de vinyle/acide crotonique/ t-butyl-4-benzoate de vinyle (65/10/25) plastifié à l'adipate de diisopropyl et ayant un taux de matière sèche de 25 % en poids (*) | 20 g |
| - Adipate de diisopropyle | 1 g |
| - Mélange de cétyl diméthicone copolyol, d'isostéarate polyglycérolé (4 moles), et de laurate d'hexyl, vendu sous la dénomination ABIL WE 09 par la société GOLDSCHMIT | |
| | 9 g |
| - Conservateurs | qs |
| - alcool éthylique | 5 g |
| - eau déminéralisée qsp | 100g |

| | |
|---|---|
| (*) La dispersion de poymère a été préparée conformément a l'exemple 1 de la demande de brevet EP-A-679384. | |

Pour préparer l'émulsion, on a chauffé l'émulsionnant, le coémulsionnant et l'agent épaississant de la phase grasse jusqu'à homogénéisation. Puis, à 65°C, on a ajouté les huiles siliconées, ainsi que les pigments puis la poudre de nylon. On a incorporé ensuite l'isododécane. Puis on a ajouté la phase aqueuse et on a réalisé l'émulsion sous agitation à l'aide d'une turbine classique. Enfin, on a ajouté l'éthanol et on a homogénéisé.

On a obtenu ainsi une émulsion fine et homogène, qui est stable en conservation pendant au moins 2 mois à température ambiante et à 45 °C. L'émulsion s'applique aisément sur le visage, en formant un film uniforme, souple et non collant. Elle présente également l'avantage de ne pas transférer sur un tissu appliqué sur la peau.

## Revendications

1. Emulsion eau-dans-huile comprenant une phase grasse comprenant une huile volatile, et une phase aqueuse comprenant un polymère filmogène en dispersion dans ladite phase aqueuse, caractérisée par le fait que ledit polymère filmogène est un copolymère comprenant au moins un monomère acide carboxylique comportant au moins une insaturation éthylénique, et comprenant au moins un ester d'acide carboxylique dont le groupement lié à l'atome d'oxygène de la fonction ester comporte au moins une insaturation éthylénique.

2. Emulsion selon la revendication 1, caractérisée par le fait que les fonctions acides carboxyliques du polymère filmogène sont neutralisées à un taux allant de 10 % à 80 % avec une base.

3. Emulsion selon l'une des revendications 1 ou 2, caractérisée par le fait que le polymère filmogène comprend en outre au moins un ester d'acide carboxylique de type acrylate.

4. Emulsion selon l'une quelconque des revendications 1 à 3 , caractérisée par le fait que le polymère filmogène est choisi dans le groupe formé par :
- les copolymères acétate de vinyle/acide crotonique
- les terpolymères acétate de vinyle/acide crotonique/néodécanoate de vinyle,
- les polymères répondant à la formule générale (I) suivante: 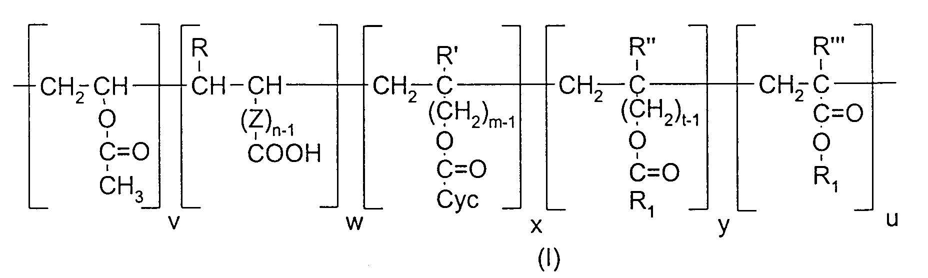 dans laquelle :
- R, R', R'' et R''', identiques ou différents, représentent un atome d'hydrogène ou un radical méthyle,
- m, n et t sont 1 ou 2,
- R1 représente un radical alkyle, linéaire ou ramifié, saturé ou insaturé ayant de 2 à 21 atomes de carbone,
- Z représente un radical divalent pris dans le groupe constitué par :
-CH2-, -CH2-O-CH2- et -CH2-O-(CH2)2-,
- Cyc représente un radical choisi parmi 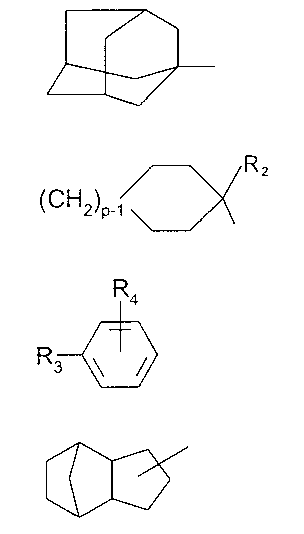 dans lesquels
- R2 représente un atome d'hydrogène ou un radical méthyle,
- R3 représente un atome d'hydrogène, un radical méthyle, éthyle, tert-butyle, éthoxy, butoxy ou dodécyloxy,
- R4 représente un atome d'hydrogène, un radical alkyle de 1 à 4 atomes de carbone ou un radical alkoxy de 1 à 4 atomes de carbone,
- p est égal à 1 ou 2
- v représente de 10 à 97% en poids,
- w représente de 3 à 20 % en poids,
- x représente de 0 à 60 % en poids,
- y représente de 0 à 40% en poids,
- u représente de 0 à 30 % en poids,
- v + w + x + y + u étant égal à 100%.

5. Emulsion selon l'une des revendications 1, 2 et 4, caractérisée par le fait que le polymère filmogène est le copolymère acétate de vinyle/acide crotonique/tertbutyl-4 benzoate de vinyle (65/10/25).

6. Emulsion selon l'une des revendications précédentes, caractérisée par le fait que le polymère filmogène en dispersion dans la phase aqueuse est présent en une teneur allant de 0,3 % à 10 % en poids par rapport au poids total de l'émulsion.

7. Emulsion selon l'une des revendications précédentes, caractérisée par le fait que l'huile volatile est choisie parmi les huiles volatiles hydrocarbonées, les huiles volatiles siliconées et leurs mélanges.

8. Emulsion selon l'une des revendications précédentes, caractérisée par le fait que l'huile volatile est choisie parmi les isoparaffines, les silicones volatiles cycliques ayant de 3 à 8 atomes de silicium et de préférence de 4 à 6, les cyclocopolymères du type diméthylsiloxane/méthylalkylsiloxane, les silicones volatiles linéaires ayant de 2 à 9 atomes de silicium, et leurs mélanges.

9. Emulsion selon l'une quelconque des revendications précédentes, caractérisée par le fait que l'huile volatile est choisie parmi l'isododécane, la cyclotétradiméthylsiloxane, la cyclopentadiméthylsiloxane, la cyclohexadiméthylsiloxane, le cyclocopolymère diméthylsiloxane/méthyloctylsiloxane, l'hexaméthyldisiloxane, l'hexyl heptaméthyltrisiloxane, l'octyl heptaméthyltrisiloxane, et leurs mélanges.

10. Emulsion selon l'une des revendications 1 à 9, caractérisée par le fait que la phase grasse comprend, en outre, au moins un corps gras non volatil à une teneur inférieure ou égale à 7 % en poids par rapport au poids total de l'émulsion.

11. Emulsion selon la revendication 10, caractérisée par le fait que la phase grasse comprend de 65 % à 99 % en poids, par rapport au poids total de la phase grasse, d' huile volatile, et de 1 % à 35 % en poids de corps gras non volatil.

12. Emulsion selon l'une des revendications 10 et 11, caractérisée par le fait que le corps gras non volatil est choisi parmi les huiles non volatiles, les corps gras pâteux, les gommes et/ou les cires végétales, minérales, animales et/ou synthétiques.

13. Emulsion selon l'une quelconque des revendications précédentes, caractérisée par le fait que la phase grasse comprend au moins 75 % en poids, par rapport au poids total de la phase grasse, d'huile volatile.

14. Emulsion selon l'une quelconque des revendications 1 à 9, caractérisée par le fait que la phase grasse comprend 100 % en poids, par rapport au poids total de la phase grasse, d'huile volatile.

15. Emulsion selon l'une quelconque des revendications précédentes, caractérisée par le fait qu'elle comprend un tensioactif ayant un HLB permettant d'obtenir une émulsion eau-dans-huile.

16. Emulsion selon la revendication 15, caractérisée par le fait que le tensioactif est choisi parmi les tensioactifs siliconés de formule (XI) suivante : 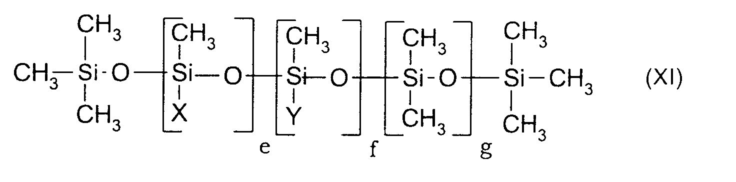 dans laquelle :
X désigne un groupement -(CH2)3-O-(C2H4O-)x(C3H6O-)y-W
W est est un atome d'hydrogène, un alkyle en C1-C16, un acyle en C1-C16,
Y est un radical alkyle en C8-C22 ou acyle en C8-C22,
g = O à 200,
e = 1 à 40,
f = 1 à 100,
le poids moléculaire du reste -(C2H4O-)ₓ(C3H6O-)_{y}-W est de 250 à 2000, x et y étant choisis de telle sorte que le rapport en poids des groupes oxyéthylène/oxypropylène soit compris entre 1000:0 et 20:80.

17. Emulsion selon l'une des revendications 15 ou 16, caractérisée par le fait que l'émulsion comprend, par rapport au poids total de l'émulsion, de 30 % à 55 % en poids de phase grasse, de 5 % à 12 % en poids de tensioactif et de 35 % à 65 % en poids de phase aqueuse.

18. Emulsion selon l'une quelconque des revendications 15 à 17, caractérisée par le fait que l'émulsion comprend de 7 % à 10 % en poids de tensioactif.

19. Composition cosmétique, pharmaceutique ou hygiénique, caractérisée par le fait qu'elle comprend une émulsion selon l'une quelconque des revendications précédentes.

20. Composition selon la revendication 19, caractérisée par le fait que la composition se présente sous forme d'un produit de maquillage tel qu'un fond de teint, un fard à joues, un fard à paupières, un eye-liner, un mascara ou un rouge à lèvres ou se présente sous forme d'une crème, d'un lait ou d'un sérum, susceptible d'être utilisée comme produit de soin.

21. Procédé de traitement non thérapeutique de la peau ou du cuir chevelu, caractérisé par le fait qu'on applique sur la peau et/ou sur le cuir chevelu une émulsion selon l'une quelconque des revendications 1 à 18, et/ou une composition selon l'une des revendications 19 et 20.

22. Procédé de maquillage de la peau et/ou du cuir chevelu, caractérisé par le fait qu'on applique sur la peau et/ou sur le cuir chevelu une émulsion selon l'une quelconque des revendications 1 à 18, et/ou une composition selon l'une des revendications 19 et 20.

23. Utilisation d'un polymère filmogène comprenant au moins un monomère acide carboxylique comportant au moins une insaturation éthylénique, au moins un ester d'acide carboxylique dont le groupement lié à l'atome d'oxygène de la fonction ester comporte au moins une insaturation éthylénique, ledit polymère filmogène étant en dispersion dans la phase aqueuse d'une émulsion eau-dans-huile comprenant une phase grasse comprenant au moins une huile volatile, dans une composition dite 'sans transfert'.

24. Utilisation d'un polymère filmogène comprenant au moins un monomère acide carboxylique comportant au moins une insaturation éthylénique, au moins un ester d'acide carboxylique dont le groupement lié à l'atome d'oxygène de la fonction ester comporte au moins une insaturation éthylénique, ledit polymère filmogène étant en dispersion dans la phase aqueuse d'une émulsion eau-dans-huile comprenant une phase grasse comprenant au moins une huile volatile, pour diminuer le transfert et/ou la migration d'une composition la comprenant.

## Claims

1. Water-in-oil emulsion comprising a fatty phase comprising a volatile oil, and an aqueous phase comprising a film-forming polymer dispersed in the said agueous phase, characterized in that the said film-forming polymer is a copolymer comprising at least one carboxylic acid monomer containing at least one ethylenic unsaturation and at least one carboxylic acid ester in which the group attached to the oxygen atom of the ester function contains at least one ethylenic unsaturation.

2. Emulsion according to Claim 1, characterized in that the carboxylic acid functions of the film-forming polymer are neutralized to a degree ranging from 10% to 80% with a base.

3. Emulsion according to either of Claims 1 and 2, characterized in that the film-forming polymer also comprises at least one carboxylic acid ester of acrylate type.

4. Emulsion according to any one of Claims 1 to 3, characterized in that the film-forming polymer is chosen from the group formed by:
- vinyl acetate/crotonic acid copolymers,
- vinyl acetate/crotonic acid/vinyl neodecanoate terypolymers,
- the polymers corresponding to the general formula (I) below: 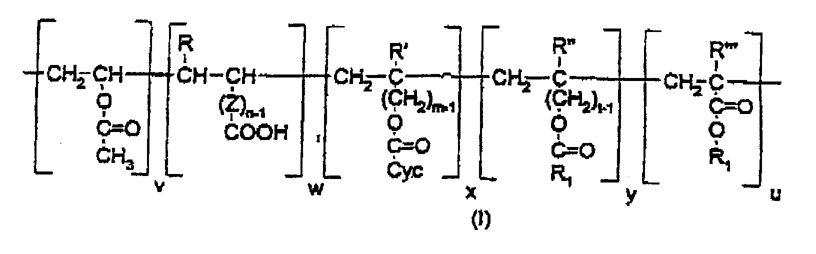 in which:
- R, R', R'' and R''', which may be identical or different, represent a hydrogen atom or a methyl radical,
- m, n and t are 1 or 2,
- R₁ represents a linear or branched, saturated or unsaturated alkyl radical having from 2 to 21 carbon atoms,
- Z represents a divalent radical taken from the group consisting of:
-CH₂-, -CH₂-O-CH₂- and -CH₂-O-(CH₂)₂-,
- Cyc represents a radical chosen from 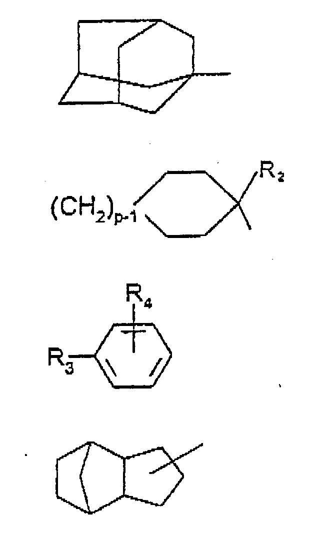 in which
- R₂ represents a hydrogen atom or a methyl radical,
- R₃ represents a hydrogen atom or a methyl, ethyl, tert-butyl, ethoxy, butoxy or dodecyloxy radical,
- R₄ represents a hydrogen atom, an alkyl radical of 1 to 4 carbon atoms or an alkoxy radical of 1 to 4 carbon atoms,
- p is equal to 1 or 2,
- v represents from 10 to 97% by weight,
- w represents from 3 to 20% by weight,
- x represents from 0 to 60% by weight,
- y represents from 0 to 40% by weight,
- u represents from 0 to 30% by weight,
- v + w + x + y + u being equal to 100%.

5. Emulsion according to one of Claims 1, 2 and 4, characterized in that the film-forming polymer is the vinyl acetate/crotonic acid/vinyl 4-tert-butylbenzoate copolymer (65/10/25).

6. Emulsion according to one of the preceding claims, characterized in that the film-forming polymer dispersed in the aqueous phase is present in a content ranging from 0.3% to 10% by weight relative to the total weight of the emulsion.

7. Emulsion according to one of the preceding claims, characterized in that the volatile oil is chosen from hydrocarbon volatile oils and silicone volatile oils, and mixtures thereof.

8. Emulsion according to one of the preceding claims, characterized in that the volatile oil is chosen from isoparaffins, volatile cyclic silicones having from 3 to 8 and preferably from 4 to 6 silicon atoms, cyclocopolymers of the dimethylsiloxane/methylalkyl-siloxane type and linear volatile silicones having from 2 to 9 silicon atoms, and mixtures thereof.

9. Emulsion according to any one of the preceding claims, characterized in that the volatile oil is chosen from isododecane, cyclotetradimethylsiloxane, cyclopentadimethylsiloxane, cyclohexadimethylsiloxane, dimethylsiloxane/methyloctylsiloxane cyclocopolymer, hexamethyldisiloxane, hexylheptamethyltrisiloxane and octylheptamethyltrisiloxane, and mixtures thereof.

10. Emulsion according to one of Claims 1 to 9, characterized in that the fatty phase also comprises at least one non-volatile fatty substance in a content of less than or equal to 7% by weight relative to the total weight of the emulsion.

11. Emulsion according to Claim 10, characterized in that the fatty phase comprises from 65% to 99% by weight, relative to the total weight of the fatty phase, of volatile oil, and from 1% to 35% by weight of non-volatile fatty substance.

12. Emulsion according to either of Claims 10 and 11, characterized in that the non-volatile fatty substance is chosen from non-volatile oils, pasty fatty substances and plant, mineral, animal and/or synthetic gums and/or waxes.

13. Emulsion according to any one of the preceding claims, characterized in that the fatty phase comprises at least 75% by weight, relative to the total weight of the fatty phase, of volatile oil.

14. Emulsion according to any one of Claims 1 to 9, characterized in that the fatty phase comprises 100% by weight, relative to the total weight of the fatty phase, of volatile oil.

15. Emulsion according to any one of the preceding claims, characterized in that it comprises a surfactant having an HLB which makes it possible to obtain a water-in-oil emulsion.

16. Emulsion according to Claim 15, characterized in that the surfactant is chosen from the silicone surfactants of formula (XI) below: 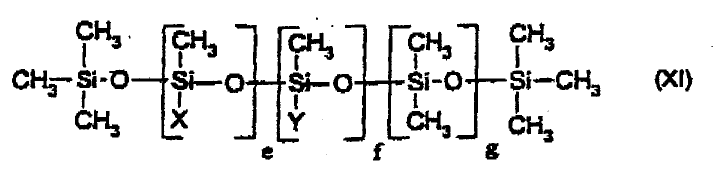 in which;
X denotes a group -(CH₂)₃-O-(C₂H₄O-)ₓ(C₃H₄O-)_{y}-W
W is a hydrogen atom, a C₁-C₁₆ alkyl or a C₁-C₁₆ acyl,
Y is a C₈-C₂₂ alkyl or C₈-C₂₂ acyl radical,
g = 0 to 200,
e = 1 to 40,
f = 1 to 100,
the molecular weight of the residue -(C₂H₄O-)ₓ(C₃H₆O-)_{y}-W is from 250 to 2000, x and y being chosen such that the weight ratio of the oxyethylene groups/oxypropylene groups is between 1000/0 and 20/80.

17. Emulsion according to either of Claims 15 and 16, characterized in that the emulsion comprises, relative to the total weight of the emulsion, from 30% to 55% by weight of fatty phase, from 5% to 12% by weight of surfactant and from 35% to 65% by weight of aqueous phase.

18. Emulsion according to any one of Claims 15 to 17, characterized in that the emulsion comprises from 7% to 10% by weight of surfactant.

19. Cosmetic, pharmaceutical or hygiene composition, characterized in that it comprises an emulsion according to any one of the preceding claims.

20. Composition according to Claim 19, characterized in that the composition is in the form of a make-up product such as a foundation, a blusher, an eye shadow, an eye liner, a mascara or a lipstick, or is in the form of a cream, a milk or a serum, which can be used as a care product.

21. Non-therapeutic process for treating the skin or the scalp, characterized in that an emulsion according to any one of Claims 1 to 18, and/or a composition according to either of Claims 19 and 20, is applied to the skin and/or the scalp.

22. Process for making up the skin and/or the scalp characterized in that an emulsion according to any one of Claims 1 to 18, and/or a composition according to either of Claims 19 and 20, is applied to the skin and/or the scalp.

23. Use of a film-forming polymer comprising at least one carboxylic acid monomer containing at least one ethylenic unsaturation, at least one carboxylic acid ester in which the group attached to the oxygen atom of the ester function contains at least one ethylenic unsaturation, the said film-forming polymer being dispersed in the aqueous phase of a water-in-oil emulsion comprising a fatty phase comprising at least one volatile oil, in a so-called "transfer-free" composition.

24. Use of a film-forming polymer comprising at least one carboxylic acid monomer containing at least one ethylenic unsaturation, at least one carboxylic acid ester in which the group attached to the oxygen atom of the ester function contains at least one ethylenic unsaturation, the said film-forming polymer being dispersed in the aqueous phase of a water-in-oil emulsion comprising a fatty phase comprising at least one volatile oil, in order to decrease the transfer and/or migration of a composition comprising it.

## Patentansprüche

1. Wasser-in-Öl-Emulsion, die eine Fettphase mit einem flüchtigen Öl und eine wäßrige Phase mit einem in der wäßrigen Phase dispergierten filmbildenden Polymer enthält,
dadurch gekennzeichnet,
daß das filmbildende Polymer ein Copolymer ist, das mindestens ein Carbonsäuremonomer mit mindestens einer ethylenischen Doppelbindung und mindestens einen Carbonsäureester enthält, dessen an das Sauerstoffatom der Estergruppe gebundene Gruppe mindestens eine ethylenische Doppelbindung aufweist.

2. Emulsion nach Anspruch 1, dadurch gekennzeichnet, daß die Carbonsäuregruppen des filmbildenden Polymers zu 10 bis 80 % mit einer Base neutralisiert wurden.

3. Emulsion nach einem der Ansprüche 1 oder 2, dadurch gekennzeichnet, daß das filmbildende Polymer ferner mindestens einen Carbonsäureester vom Acrylattyp enthält.

4. Emulsion nach einem der Ansprüche 1 bis 3, dadurch gekennzeichnet, daß das filmbildende Polymer ausgewählt ist unter:
- den Vinylacetat/Crotonsäure - Copolymeren,
- den Vinylacetat/Crotonsäure/Vinylneodecanoat - Terpolymeren,
- den Polymeren mit der folgenden allgemeinen Formel (I): 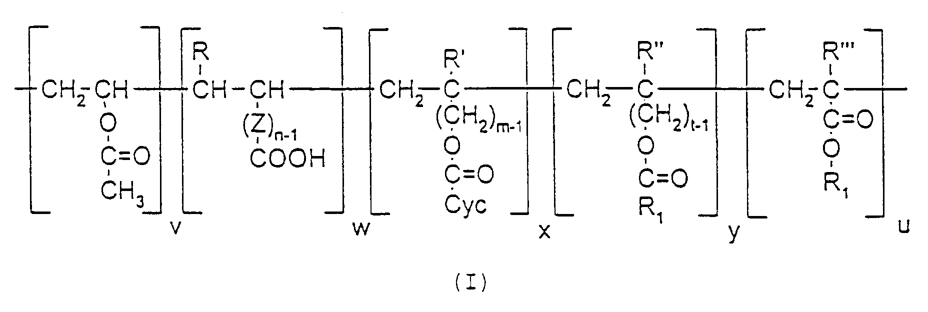 worin bedeuten:
- R, R', R'' und R''', die identisch oder voneinander verschieden sind, Wasserstoff oder Methyl,
- m, n und t 1 oder 2,
- R₁ eine geradkettige oder verzweigte, gesättigte oder ungesättigte Alkylgruppe mit 2 bis 21 Kohlenstoffatomen,
- Z eine zweiwertige Gruppe, die ausgewählt ist unter: -CH₂-, -CH₂-O-CH₂- und -CH₂-O-(CH₂)₂-,
- Cyc eine Gruppe, die ausgewählt ist unter 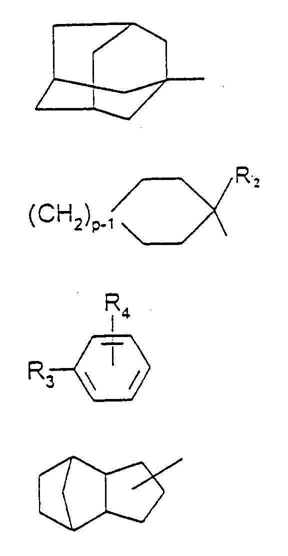 worin bedeuten:
- R₂ Wasserstoff oder Methyl,
- R₃ Wasserstoff, Methyl, Ethyl, *tert*.-Butyl, Ethoxy, Butoxy oder Dodecyloxy,
- R₄ Wasserstoff, eine Alkylgruppe mit 1 bis 4 Kohlenstoffatomen oder eine Alkoxygruppe mit 1 bis 4 Kohlenstoffatomen,
- p 1 oder 2,
- v 10 bis 97Gew.-%,
- w 3 bis 20 Gew.-%,
- x 0 bis 60 Gew.-%,
- y 0 bis 40 Gew.-%,
- u 0 bis 30 Gew.-%,
- wobei v + w + x + y + u 100 % ergeben.

5. Emulsion nach einem der Ansprüche 1, 2 und 4, dadurch gekennzeichnet, daß das filmbildende Polymer das Vinylacetat/Crotonsäure/Vinyl-4-*tert*.-Butyl-Benzoat (65/10/25) - Copolymer ist.

6. Emulsion nach einem der vorhergehenden Ansprüche, dadurch gekennzeichnet, daß das in der wäßrigen Phase dispergierte filmbildende Polymer in einem Mengenanteil im Bereich von 0,3 bis 10 Gew.-%, bezogen auf das Gesamtgewicht der Emulsion, vorliegt.

7. Emulsion nach einem der vorhergehenden Ansprüche, dadurch gekennzeichnet, daß das flüchtige Öl unter den flüchtigen Kohlenwasserstoffölen, den flüchtigen Siliconölen und deren Gemischen ausgewählt ist.

8. Emulsion nach einem der vorhergehenden Ansprüche, dadurch gekennzeichnet, daß das flüchtige Öl unter den Isoparaffinen, den cyclischen flüchtigen Siliconen mit 3 bis 8 Siliciumatomen und vorzugsweise 4 bis 6 Siliciumatomen, den Cyclocopolymeren vom Typ Dimethylsiloxan/Methylacrylsiloxan, den geradkettigen flüchtigen Siliconen mit 2 bis 9 Siliciumatomen und deren Gemischen ausgewählt ist.

9. Emulsion nach einem der vorhergehenden Ansprüche, dadurch gekennzeichnet, daß das flüchtige Öl unter Isododecan, Cyclotetradimethylsiloxan, Cyclopentadimethylsiloxan, Cyclohexadimethylsiloxan, dem Dimethylsiloxan/Methyloctylsiloxan-Cyclocopolymer, Hexamethyldisiloxan, Hexylheptamethyltrisiloxan, Octylheptamethyltrisiloxan und deren Gemischen ausgewählt ist.

10. Emulsion nach einem der Ansprüche 1 bis 9, dadurch gekennzeichnet, daß die Fettphase ferner mindestens eine nichtflüchtige Fettsubstanz in einem Mengenanteil von höchstens 7 Gew.-%, bezogen auf das Gesamtgewicht der Emulsion, enthält.

11. Emulsion nach Anspruch 10, dadurch gekennzeichnet, daß die Fettphase 65 bis 99 Gew.-% flüchtiges Öl und 1 bis 35 Gew.-% nichtflüchtige Fettsubstanz, bezogen auf das Gesamtgewicht der Fettphase, enthält.

12. Emulsion nach einem der Ansprüche 10 und 11, dadurch gekennzeichnet, daß die nichtflüchtige Fettsubstanz unter den nichtflüchtigen Ölen, den pastösen Fettsubstanzen, den Gummen und/oder Wachsen pflanzlichen, mineralischen, tierischen und/oder synthetischen Ursprungs ausgewählt ist.

13. Emulsion nach einem der vorhergehenden Ansprüche, dadurch gekennzeichnet, daß die Fettphase mindestens 75 Gew.-% flüchtiges Öl, bezogen auf das Gesamtgewicht der Fettphase, enthält.

14. Emulsion nach einem der Ansprüche 1 bis 9, dadurch gekennzeichnet, daß die Fettphase 100 Gew.-% flüchtiges Öl, bezogen auf das Gesamtgewicht der Fettphase, enthält.

15. Emulsion nach einem der vorhergehenden Ansprüche, dadurch gekennzeichnet, daß sie ein grenzflächenaktives Mittel enthält, das einen solchen HLB-Wert aufweist, daß eine Wasser-in-Öl-Emulsion hergestellt werden kann.

16. Emulsion nach Anspruch 15, dadurch gekennzeichnet, daß der grenzflächenaktive Stoff unter den grenzflächenaktiven Siliconen der folgenden Formel (XI) ausgewählt ist: 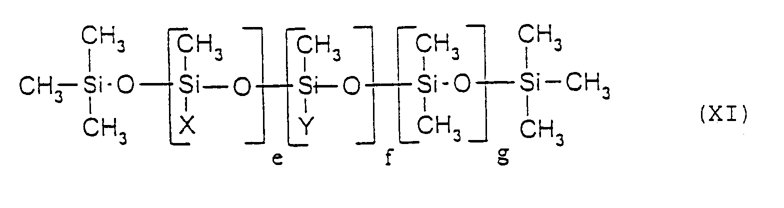 worin bedeuten:
X eine Gruppe -(CH₂)₃-O-(C₂H₄O-)ₓ(C₃H₆O-)_{y}-W
W Wasserstoff, C₁₋₁₆-Alkyl oder C₁₋₁₆-Acyl,
Y C₈₋₂₂-Alkyl oder C₈₋₂₂-Acyl,
g = 0 bis 200,
e = 1 bis 40,
f = 1 bis 100,
wobei das Molekulargewicht der Gruppe
-(C₂H₄O-)ₓ(C₃H₆O-)_{y}-W 250 bis 2000 beträgt und x und y so ausgewählt sind, daß das auf das Gewicht bezogene Verhältnis der Gruppen Ethylenoxid/Propylenoxid im Bereich von 1000:0 bis 20:80 liegt.

17. Emulsion nach einem der Ansprüche 15 oder 16, dadurch gekennzeichnet, daß die Emulsion 30 bis 55 Gew.-% Fettphase, 5 bis 12 Gew.-% grenzflächenaktives Mittel und 35 bis 65 Gew.-% wäßrige Phase, bezogen auf das Gesamtgewicht der Emulsion, enthält.

18. Emulsion nach einem der Ansprüche 15 bis 17, dadurch gekennzeichnet, daß die Emulsion 7 bis 10 Gew.-% grenzflächenaktives Mittel enthält.

19. Kosmetische, pharmazeutische oder hygienische Zusammensetzung, dadurch gekennzeichnet, daß sie eine Emulsion nach einem der vorhergehenden Ansprüche enthält.

20. Zusammensetzung nach Anspruch 19, dadurch gekennzeichnet, daß die Zusammensetzung in Form eines Produkts zum Schminken, wie als Make-up, Wangenrouge, Lidschatten, Eyeliner, Mascara oder Lippenstift, oder in Form einer Creme, einer Milch oder eines Serums vorliegt, die als Produkte zur Pflege verwendet werden können.

21. Verfahren zur nicht therapeutischen Behandlung der Haut oder der Kopfhaut, dadurch gekennzeichnet, daß auf die Haut und/oder die Kopfhaut eine Emulsion nach einem der Ansprüche 1 bis 18 und/oder eine Zusammensetzung nach einem der Ansprüche 19 und 20 aufgetragen wird.

22. Verfahren zum Schminken der Haut und/oder der Kopfhaut, dadurch gekennzeichnet, daß auf die Haut und/oder die Kopfhaut eine Emulsion nach einem der Ansprüche 1 bis 18 und/oder eine Zusammensetzung nach einem der Ansprüche 19 und 20 aufgetragen wird.

23. Verwendung eines filmbildenden Polymers, das mindestens ein Carbonsäuremonomer mit mindestens einer ethylenischen Doppelbindung und mindestens einen Carbonsäureester enthält, dessen direkt an das Sauerstoffatom der Estergruppe gebundene Gruppe mindestens eine ethylenische Doppelbindung aufweist, wobei das filmbildende Polymer in der wäßrigen Phase einer Wasser-in-Öl-Emulsion dispergiert ist, die eine Fettphase aufweist, die mindestens ein flüchtiges Öl enthält, in einer Zusammensetzung ohne Transfer.

24. Verwendung eines filmbildenden Polymers, das mindestens ein Carbonsäuremonomer mit mindestens einer ethylenischen Doppelbindung und mindestens einen Carbonsäureester enthält, dessen direkt an das Sauerstoffatom der Estergruppe gebundene Gruppe mindestens eine ethylenische Doppelbindung aufweist, wobei das filmbildende Polymer in der wäßrigen Phase einer Wasser-in-Öl-Emulsion dispergiert ist, die eine Fettphase aufweist, die mindestens ein flüchtiges Öl enthält, um den Transfer und/oder die Migration einer diese enthaltenden Zusammensetzung zu vermindern.
